# EUROPEAN PATENT SPECIFICATION

(11) **EP 3 027 752 B1**
(45) Date of publication and mention of the grant of the patent: **08.08.2018**
(21) Application number: 14819385.7
(22) Date of filing: 27.06.2014
(51) Int. Cl.: C12N 15/11, C12N 15/63, C12N 15/09, C12N 15/81

(54) **SIGNAL SEQUENCE FOR PROTEIN EXPRESSION IN PICHIA PASTORIS**
SIGNALSEQUENZ FÜR PROTEINEXPRESSION IN PICHIA PASTORIS
SÉQUENCE SIGNAL POUR L'EXPRESSION DE PROTÉINE DANS PICHIA PASTORIS

(30) Priority: 01.07.2013 IN 2905CH2013
(43) Date of publication of application: 08.06.2016
(73) Proprietor: Biocon Limited, Bengaluru 560100 (IN)
(72) Inventor: GOVINDAPPA, Nagaraj, Bengaluru 562130 (IN); SREENIVAS, Suma, Bengaluru 560003 (IN); PERIYASAMY, Sankar, Tirupur 638108 (IN); DIVAKAR, Srividya, Bengaluru 560036 (IN)
(74) Representative: Michalski Hüttermann & Partner Patentanwälte mbB
(86) International application number: PCT/IB2014/062682
(87) International publication number: WO 2015/001460

(56) References cited:
- WO-A1-2011/106389
- US-A- 5 965 386
- GOVINDAPPA NAGARAJ ET AL: "A New Signal Sequence for Recombinant Protein Secretion in Pichia pastoris", March 2014 (2014-03), JOURNAL OF MICROBIOLOGY AND BIOTECHNOLOGY, VOL. 24, NR. 3, PAGE(S) 337-345, XP002763196, ISSN: 1017-7825(print) * the whole document *
- JOAN LIN CEREGHINO ET AL.: 'Heterologous protein expression in the methylotrophic yeast Pichia pastoris' FEMS MICROBIOLOGY REVIEWS vol. 24, no. 1, 2000, pages 45 - 66, XP002261542

## Description

### RELATED APPLICATION

This application claims benefit of Indian Provisional Application No. 2905/CHE/2013 filed on July 1, 2013.

### FIELD OF INVENTION

The present disclosure relates to a signal sequence of 18 amino acids from a unique *Pichia pastoris* protein. Further the invention discloses use of signal sequence for the expression of heterologous protein in *Pichia pastoris.*

### BACKGROUND AND PRIOR ART OF THE INVENTION

Signal sequences are N-terminal extensions of nascent polypeptide chains that mediate protein targeting to the membrane of the endoplasmic reticulum (ER). They are on average 16 to 30 amino acid residues in length comprising a characteristic tripartite structure: (1) a hydrophilic, usually positively charged n-region, (2) a central hydrophobic h-region of 5-15 residues and (3) a c-region with the cleavage site for signal peptidase (SPase). The consensus cleavage site consists of amino acids with short side chains at the -1 and no charged amino acid residues at the -3 position. Besides these common features, signal sequences can be quite different in a. a sequence length and mode of function.

Signal peptides control the entry of virtually all proteins to the secretory pathway, both in eukaryotes and prokaryotes. They comprise the N-terminal part of the amino acid (aa) chain and are cleaved off while the protein is translocated through the membrane. The presence of N-terminal signal sequences leads to strong stimulation and expression of recombinant proteins. The proteins secreted from eukaryotic cells require an N-terminal signal peptide.

A signal sequence means a sequence included at the beginning of the coding sequence of a protein to be expressed on the surface of a cell. This sequence encodes a signal peptide, N-terminal to the mature polypeptide, that directs the host cell to translocate the polypeptide.

Currently the secretory expression in *Pichia pastoris* makes use of the pre-pro mat alpha factor sequence from *Saccharomyces cerevisiae* and the dibasic amino acid cleavage site (KR) at the end of signal sequence. This efficiently processed by the Kex2 protease (Kex2p) in the host resulting in the secretion of mature proteins to the medium with desired N-terminal amino acid. However, the proteins which have one or more internal dibasic amino acids such as KR and RR in the coding region cannot be expressed using this signal sequence as the protein gets fragmented.

Hence, there exists a need in the art to produce a full length heterologous protein in the medium having dibasic amino acid cleavage site (KR and RR). The inventors of the present invention have found that the identified signal sequence will be very useful to express the heterologous protein having internal Kex2p cleavage sites using Kex2p disrupted *Pichia pastoris* strain.

### STATEMENT OF THE INVENTION

The present disclosure relates to a signal sequence of 18 amino acids from a unique *Pichia pastoris* protein. Further the invention discloses use of signal sequence for the expression of heterologous protein in *Pichia pastoris.*

In an embodiment of the present invention said signal sequence of 18 amino acids is identified and isolated from *Pichia pastoris.*

In another embodiment of the present disclosure, analysis of extracellular protein profile of all the inactivated strains revealed that the ∼ 30 KDa proteins being secreting into the medium from the Kex2p disrupted *Pichia pastoris.*

In still another embodiment of the present disclosure the protein of a single band within a gel after electrophoretic separation of secreted proteins was subjected to N-terminal sequencing by Edman degradation method and the amino acids sequence derived are, "DQTFGVLLIRSG".

In another embodiment of the present disclosure, the 18 amino acid signal sequence was not present at the N-terminus of the secreted protein and this protein is secreting only upon Kex2p disrupted *Pichia pastoris.*

In an embodiment of the present disclosure, 18 amino acid at the N-terminus from *P. pastoris* were identified in by the following process;
a) cloning the 30 KDa protein under the control of strong AOX1 promoter inframe with this novel signal sequence and secreted protein in wild type *P. pastoris* strain;
b) expressing various constructs and transforming into *P. pastoris* strain to evaluate and characterize the signal sequence.
c) inducing the resulting strains with methanol using the shake flask experiments.

The results proved that the 18 amino acid signal peptide indeed has a potential to secrete the heterologous proteins efficiently into the medium and that this signal sequence is cleaved of independently without the use of Kex2p cleavage sites.

In still another embodiment of the present disclosure, the signal sequence is more useful in expressing the recombinant proteins with internal di-basic amino acids such as KR and RR Kex2p cleavage sites.

### BRIEF DESCRIPTION OF THE ACCOMPANYING FIGURES

The features of the present disclosure will become more fully apparent from the following description taken in conjunction with the accompanying drawings. Understanding that the drawings depict only several embodiments in accordance with the disclosure and are therefore not to be considered limiting of its scope, the disclosure will be described with additional specificity and detail through use of the accompanying drawings:

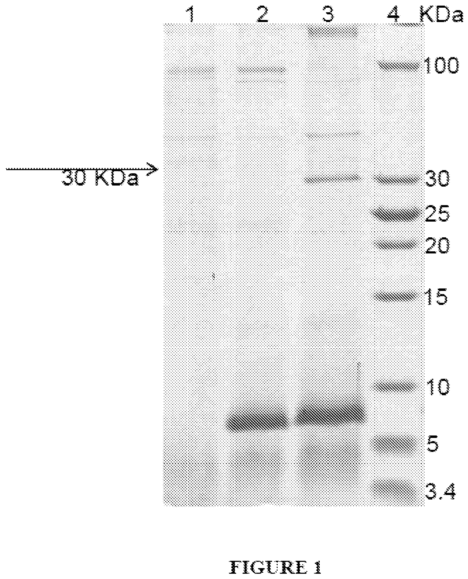

**Figure 1**: Secretion of 30 KDa DDDK protein in the gene inactivated host.
**Figure 2A****:** The construct were develop to evaluate the novel signal sequence properties of the 18 amino acids of N-terminus of DDDK protein under the regulatory control of AOX1 promoter.
**Figure 2B (i)**: Analysis of DDDK protein expression in *P. pastoris* wild type and PMT1 gene knock out host
**Figure 3****:** The amino acid sequence of the 30 KDa DDDK protein identified by blasting against the *P. pastoris* database.
**Figure 4A****:** The 30 KDa DDDK protein secreted into the medium when expressed the protein under the regulatory control of AOX1 promoter in wild type *P. pastoris*; and
**Figure 4B****:** Comparison of 30 KDa DDDK protein secretion when inserted Kex2p cleavage site after 18 amino acid signal sequence.
**Figure 5****:** The Western blot analysis of the 30 KDa DDDK protein to identify whether any protein accumulates intracellularly due to non-processing of signal sequence.
**Figure 6A****:** The porcine Carboxypeptidase B expressed using DDDK signal sequence has secreted into the medium as efficiently as one secreted with Mata signal sequence.
**Figure 6B****:** The *Erythrina* Trypsin Inhibitor (ETI) expressed using DDDK signal sequence has secreted into the medium is comparable to the one secreted with Mata signal sequence **Figure 7**: The primers used for genetic manipulation of the strains developed.

### DETAILED DESCRIPTION OF THE INVENTION

The present disclosure relates to a signal sequence of 18 amino acids from a unique *Pichia pastoris* protein. Further the invention discloses use of signal sequence for the expression of heterologous protein in *Pichia pastoris.*

In an embodiment of the present invention said amino acid is identified and isolated from *Pichia pastoris.*

In another embodiment of the present disclosure, analysis of extracellular protein profile of all the inactivated strains revealed that the ∼ 30 KDa protein (SEQ ID NO: 4) being secreting into the medium from the Kex2p disrupted *Pichia pastoris.*

In still another embodiment of the present disclosure, the protein band representing the ∼ 30 KDa protein was subjected to N-terminal sequencing by Edman degradation method and the amino acids sequence derived is DQTFGVLLIRSG (SEQ ID NO: 3).

In another embodiment of the present disclosure the 18 amino acid at the N-terminus were not part of the secreted protein and this protein is secreting into the medium from the Kex2p disrupted *Pichia pastoris.*

In an embodiment of the present disclosure; 18 amino acid at the N-terminus from P.pastoris were identified in by the following process;
a) First cloned this 30 KDa protein under the control of strong AOX1 promoter inframe with this novel signal sequence and secreted protein in wild type P.pastoris strain.
b) Various expressions construct and transformed into P.pastoris strain to evaluate and characterize the signal sequence.
c)the resulting strains were induced with methanol using the shake flask experiments and results proved that the 18 amino acid signal peptide indeed has a potential to secrete the heterologous proteins efficiently into the medium and this signal sequence is cleaved of independently without the use of Kex2p cleavage sites.

In still another embodiment of the present disclosure; signal sequence is more useful in expressing the recombinant proteins with internal di-basic amino acids such as KR and RR Kex2p cleavage sites.

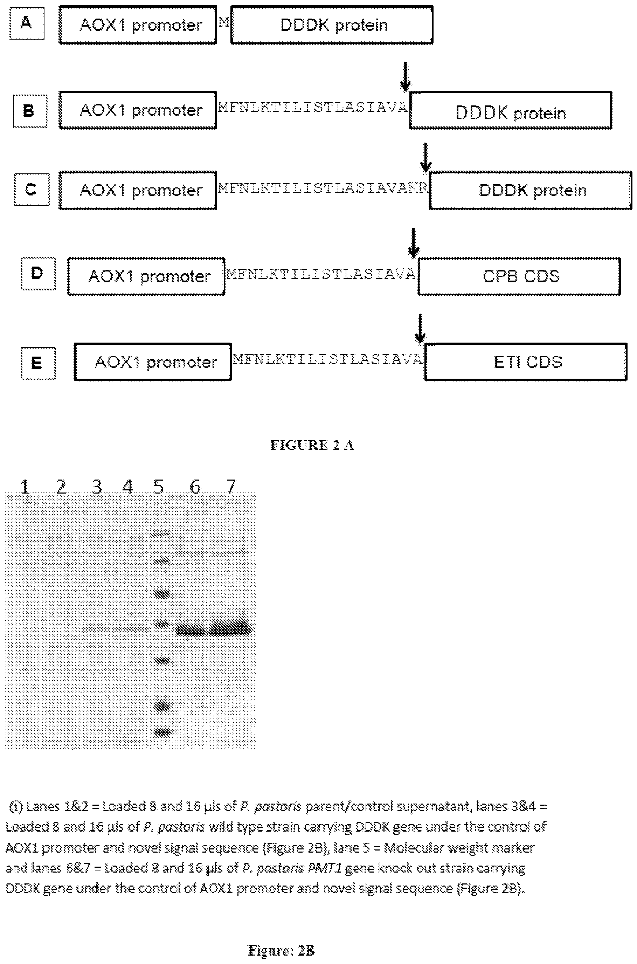

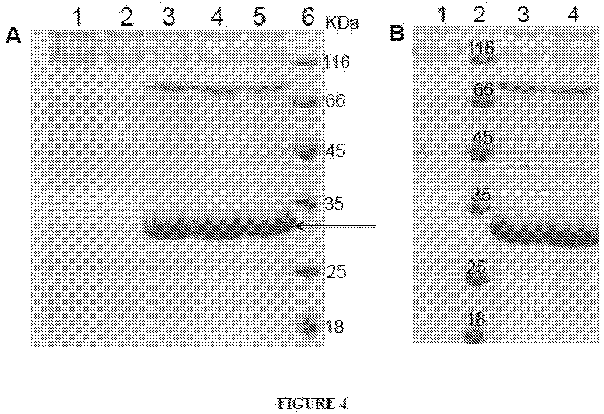

In an embodiment of the present invention; the signal sequence of DDDK protein can be used for the expression of recombinant protein. The DDDK protein along with its signal sequence under AOX1 promoter (Figure 2B) and successfully secreted the protein in wild type *P. pastoris* strain (Figure 4).

According to the most significant aspects of the present disclosure; a)the 18 amino acid signal sequence is able to secrete the proteins into the medium, b)the secretion of protein is independent of the Kex2p cleavage after signal sequence and c) the strong promoter is able secrete same protein in wild type *P. pastoris* also.

In another embodiment of the present disclosure ; *P. pastoris* strain BICC 9450 [9] and its derivative BICC 9682 (his⁻) hosts were used for the current studies and modified pMBL 208 expression vector which provides the promoter and terminator sequences of the *P. pastoris* AOX1 gene.

In still another embodiment of the present disclosure; The *S. cerevisiae* Matα signal sequence was replaced with "MFNLKTILISTLASIAVA" signal sequence for secretion of proteins into the medium. The *E. coli* strain DH5α was used for routine cloning and propagation of plasmids.

In yet another embodiment of the present disclosure; Yeast extract-peptone-dextrose (YPD) used in medium containing 10 g/liter yeast extract, 20 g/liter peptone and 20 g/liter dextrose was used for routine growth and subculturing of *Pichia* strains. YNBD medium used for selection contained 13.4 g/liter yeast nitrogen base (YNB) without amino acids and 20 g/liter dextrose (D). Luria broth/agar was used for culturing E. coli.

As used herein, "heterologous" means not naturally contiguous. For example, a yeast secretion signal peptide sequence and a human polypeptide sequence are heterologous because the two sequences are not naturally contiguous.

In another embodiment of the present disclosure, the most preferred host cells are methylotrophic yeasts. Strains of a methylotrophic yeast which can be modified using the present disclosure include, but are not limited to yeast strains capable of growing on methanol, such as yeasts of the genera *Pichia, Candida, Hansenula,* or *Torulopsis.* Preferred methylotrophic yeasts are of the genus *Pichia.* Methylotrophic yeast strains which can be modified using the present methods also include those methylotrophic yeast strains which have been engineered to express one or more heterologous proteins of interest.

In another embodiment of the present disclosure, the term "promoter" is used in the normal sense of the art, e.g. an RNA polymerase binding site.

The term "promoter" is used in the normal sense of the art, e.g. an RNA polymerase binding site.

The term "vector" includes expression vectors, replicable vectors, transformation vectors and shuttle vectors, including vector combinations thereof.

The term "expression vector" means a construct capable of in-vivo or in-vitro expression. Preferably the expression vector is incorporated in the genome of the organism.

The term "incorporated" preferably covers stable incorporation into the genome.

As used herein the term "expression" refers to a process by which a polypeptide is produced based on the nucleic acid sequence of a gene. The process includes both transcription and translation.

The terms "polypeptide," "peptide" and "protein" are used interchangeably herein to refer to a polymer of amino acid residues of any length.

The term "polypeptide of interest" refers to any polypeptide that may be expressed in a host cell as disclosed herein, preferably in a strain of *Pichia pastoris* as disclosed herein. The polypeptide of interest may be purified from the culture medium said host cell is or has been cultivated in during expression of said polypeptide of interest. The polypeptide of interest may for example be be an enzyme, a hormone, a ligand for a receptor or an antibody.

The term "construct", as used herein, refers to any recombinant polynucleotide molecule. Examples of constructs may be a plasmid, a cosmid, a virus, an autonomously replicating polynucleotide molecule, a phage, or a linear or circular single-stranded or double-stranded DNA or RNA polynucleotide molecule, derived from any source, capable of genomic integration or autonomous replication, comprising a polynucleotide molecule where one or more polynucleotide molecule(s) has been linked in a functionally operative manner, i.e., operably linked

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH).

As used herein, the term "signal sequence" refers to a sequence of amino acid residues bound at the amino terminus of a nascent protein during protein translation, which when recognized by the signal recognition particle results in the transport of the nascent protein to the organelle of destination. During protein translation, a specific protein is being produced through the action of mRNA and ribosome. A signal sequence comprised of amino acid residues may be produced eventually and bound as part of the growing protein.

The signal sequence is like a "flag" bound at the amino terminus of the emerging protein. This "flag" signals the transport mechanism of the cell to prompt them as to where the emerging protein should go. This "flag" is recognized by signal recognition particle (SRP) located freely in the cytoplasm or attached to the ribosome.

The SRP interacts with the signal sequence, which leads to the transient arrest of translation. At this time, the protein (not yet fully formed) is transported to the destination organelle, e.g. endoplasmic reticulum especially when the protein is for secretion. Upon docking to the membrane of the endoplasmic reticulum the SRP detaches and this leads to the continuation of protein translation, and eventually to the maturation of protein in which the signal sequence is cleaved by the signal peptidase.

The invention is further illustrated by the following examples. The following examples are provided for illustrative purposes only and are not intended to limit the scope of the invention.

### EXPERIMENTAL EXAMPLES

### Example 1:

### Construction of the Expression constructs for secretory expression in P. pastoris

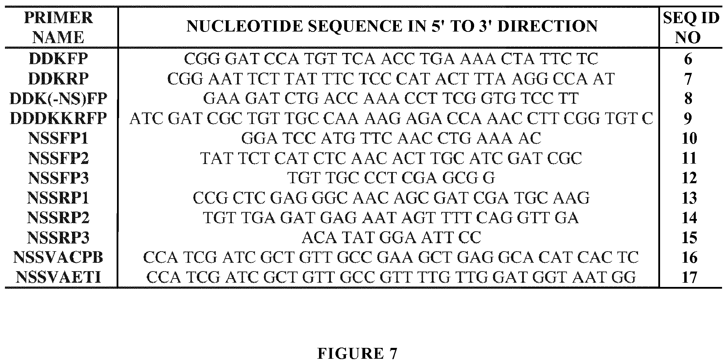

The gene corresponding to the DDDK protein (SEQ ID NO: 4) was amplified along with signal sequence using primers DDKFP (SEQ ID NO: 6) and DDKRP (SEQ ID NO: 7) (Figure 7) and *P*. *pastoris* genomic DNA template. The 870 bps PCR product was cloned into pTZ57R vector, sequence verified. The gene excised using BamHI and EcoRI sites and subcloned into pMBLNSS208 vector in BamHI and EcoRI restriction sites to obtain DDK/pMBLNSS208 vector which replaces the Mata signal sequence. The DDDK gene is under the regulatory control of AOX1 promoter (SEQ ID NO: 19) (Fig. 2B). The DDDK protein ORF without the signal sequence (SEQ ID NO: 5) was amplified and cloned similarly to have a control for the above (Fig. 2A). The forward primer used was DDK(-SS)FP (SEQ ID NO: 8) in place of DDKFP. These two constructs were used to study whether the strong promoter can lead to secretion of this protein in the wild type *Pichia.* The constructs Fig.2C is similar Fig. 2B but with the Kex2p site (KR) at the end of signal sequence. This was developed to evaluate the level of secretion with and without Kex2p cleavage site.

### Example 2:

### Synthesis of Signal sequence and Development of CPB and ETI clones

The signal sequence was synthesized using the primers NSSFP1 (SEQ ID NO: 10), NSSFP2 (SEQ ID NO: 11), NSSFP3 (SEQ ID NO: 12), NSSRP1 (SEQ ID NO: 13), NSSRP2 (SEQ ID NO: 14) and NSSRP3 (SEQ ID NO: 15) (Figure 7). The primers were phosphorylated by treating with T4 polynucleotide kinase, annealed and ligated. The full length signal sequence was amplified from the ligated template using NSSFP1 (SEQ ID NO: 10) and NSSRP1 (SEQ ID NO: 13). The PCR product was cloned into pTZ57R vector and sequence verified. The signal sequence was cloned into pMBL208 vector to replace the Mata signal sequence.

The CPB codsing sequence (SEQ ID NO: 20) and the ETI coding sequence (SEQ ID NO: 24) were amplified using the forward primers NSSVACPB (SEQ ID NO: 16) and NSSVAETI (SEQ ID NO: 17) and pPIC9K reverse primer (SEQ ID NO: 18) from the plasmid CPB/pPIC9K and ETI/pPIC9K which were expressed previously in our laboratory. The forward primer has the ClaI restriction site. The PCR product was digested with ClaI and EcoRI and cloned into the DDK/pMBLNSS208 vector in the identical sites to yield the constructs Fig. 2D and Fig. 2E. These constructs will not have amino acids 'KR', the Kex2p cleavage sites at the N-terminus of mature coding region.

### Example 3:

### Transformation of Pichia pastoris

The plasmids constructs were digested with SacI and transformed into *P. pastoris* (his⁻) strain. Transformation was carried out by electroporation of freshly prepared competent cells in 0.2 cm cuvettes. The pulses were delivered by Gene Pulser (BioRad) at 1500 V, 25 µF, and 200 Ω. The electroporated cells were allowed to recover for 1hour in 1M Sorbitol at 30°C and then spread on to YNBD agar plates. The resulting transformants were selected on YPD plates, containing 0.5 mg/ml of G418 concentrations to identify multicopy clones. Several clones that were viable at 0.5 mg/ml concentration were tested in small scale induction experiments. The proteins secreted to the extracellular medium were analyzed on 10% SDS-PAGE.

### Example 4:

### Induction of the proteins expression in P. pastoris

The clones were grown on 10 ml YNB medium (1.34% yeast nitrogen base w/o amino acids and 2% dextrose) overnight. It was sub cultured into 50 ml BMGY (1% yeast extract, 2% peptone, 1.34% YNB, 100 mM potassium phosphate at pH 6.0 and 1% glycerol) with 0.5 OD₆₀₀. This culture was incubated at 30°C for 48 h with shaking at 220 rpm. The cells were harvested by centrifugation at 5000 rpm for 5 min at room temperature and resuspended in induction medium (1% yeast extract, 2% peptone, 1.34% YNB and 100 mM potassium phosphate at pH 6.0). Methanol (2%) was added to the culture medium to give a final concentration of 0.5% methanol as an inducer. Samples were collected every day and analyzed by SDS-PAGE.

A construct shown in Fig 2B was transformed into *P. pastoris* wild type and *PMT1* gene inactivated *P. pastoris* (US 2012/20309935) and the clones were shortlisted as described in example 3 and 4. The DDDK protein secreted to the medium was analyzed on 12% SDS PAGE.

**Summary**: It is very clear from the gel picture that the DDDK protein secretion is enhanced when expressed in *PMT1* gene knock out host as it is evident from lanes 6 and 7 as compared to corresponding lanes 3 and 4.

### Example 5:

### Western blotting

Western blotting was carried out as per the method described by Towbin with few modifications. The proteins samples were separated on 12% SDS-PAGE and then transferred to the Nitrocellulose sheet, 0.2 µM (Bio-Rad Laboratories, USA) as described above. After transfer, the membrane was soaked in 1% BSA in Tris NaCl buffer (20 mM Tris pH 7.2, 150mM glycine) and incubated overnight to block the non-specific binding. The membrane was washed with wash buffer (20 mM Tris pH 7.2, 150mM glycine, 0.05% Tween-20) thoroughly. The membrane was probed with an anti-DDK monoclonal antibody (OriGene Technologies, Inc, Rockville, MD 20850) at 1:100 dilution with Tris NaCl buffer containing 0.1% BSA. The DDK protein was identified with a polyclonal Sheep anti-mouse IgG-HRP conjugate (product code: NA931, GE Health care, UK Limited) using TMB substrate.

### Example 6:

### Amino acid analysis and identification of signal sequence

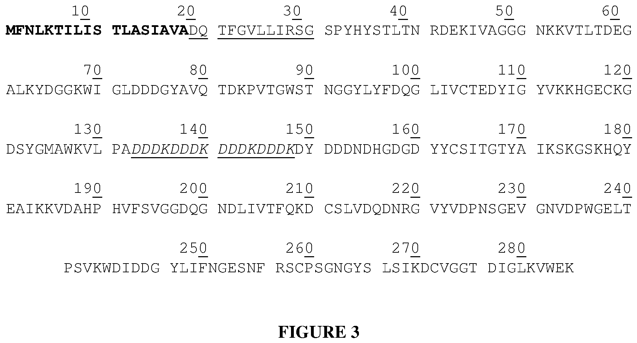

The 30 KDa protein secreted in the PMT1 gene inactivated *P. pastoris* strain was immobilized onto Sequi-blot-PVDF membrane and submitted for N-terminal a a sequencing at ProSeq. Inc. USA. The first 12 aa were found to be "DQTFGVLLIRSG" (SEQ ID NO: 3). This sequence was blasted against the NCBI database Blastp program to find out the probable homologous protein. It resulted in the identification of a 289 aa containing hypothetical protein in the *P. pastoris* GS115 genome database (SEQ ID NO: 4). There are 18 aa at the N-terminal of the protein (SEQ ID NO: 1) were acting as a signal sequence (Fig. 3) and were cleaved off during the secretion of protein to the mature protein into the medium. The theoretical mass of this protein is found to be 31.66 KDa with its pI of 4.44 when calculated using ExPASY protein parameter tool. It is highly acidic protein with four 'DDDK' aa repeats at a stretch in the primary a sequence.

### Example 7:

### Secretion of DDDK protein under AOX1 promoter in wild type P. pastoris

In order to evaluate whether this protein will come out if fused with strong promoter AOX1 (SEQ ID NO: 19). The 870 bps complete DDDK ORF which includes the coding sequence of 18 aa signal peptide was cloned under the regulatory control of AOX1 promoter (Fig.2B). The construct was transformed into the *P. pastoris* wild type strain. The clones obtained were screened for the secretory production of the DDDK protein and the good secretory expression of DDDK protein in the wild type *P. pastoris* strain (Fig.4). This indicates that the promoter of the native protein is indeed very weak and may not be able sufficiently produce the sufficient amount of protein to secrete into the medium, although it has a potential signal sequence at the N-terminus. The results shown in Fig. 4B indicates that the signal sequence can secrete the protein independent of Kex2p cleavage site as evident from the similar band intensity in lane 3 and 4.

### Example 8:

### Identification of production of DDDK protein using antibody in wild type

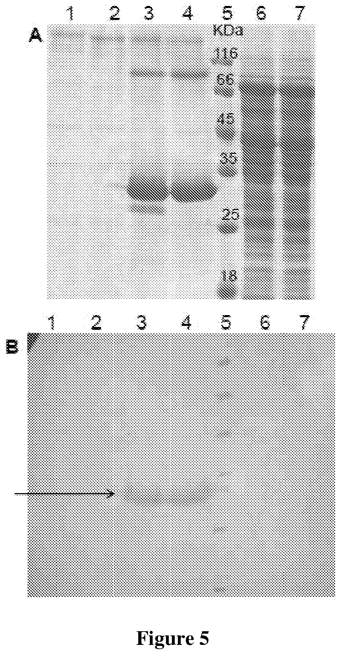

Western blot experiment using a monoclonal antibody that bind to proteins which have DDDK motif was carried out. The protein was not detected in the supernatants of *Pichia* wild type strain and *Pichia* strain carrying DDDK protein under the regulatory control of AOX1 promoter. It was seen in any band corresponds to DDK protein in the wild type *Pichia* strain (lane 1, Fig. 5) indicating that the protein is being secreted only from the Kex2p disrupted *Pichia pastoris.* It was also noticed that there was no band observed in the intracellular fraction of *Pichia* proteins indicating that all the protein expressed has successfully secreted to the medium with use of this novel DDDK signal sequence.

### Example 9:

### Secretion of CPB and ETI using DDK protein signal sequence

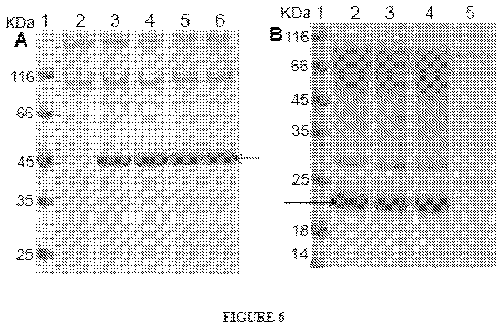

To show whether the signal sequence functions if we use for secreting heterologous proteins, we have developed the constructs having CPB and ETI coding sequences (SEQ ID NOs: 20 and 24) fused in frame with this signal sequence (SEQ ID NO: 2) (Figure 2A -D and E) without the Kex2p cleavage sites. The resulting nucleic acid sequences are represented by SEQ ID NOs: 22 and 26. The constructs were transformed into the *P. pastoris* wild type strain. The clones obtained were screened for the secretory production of these proteins. We found the good secretory expression of CPB (SEQ ID NO: 21) and ETI (SEQ ID NO: 25) proteins which are equivalent the proteins produced using mat-α signal sequence (Figure. 6).

The present invention comprises the following embodiments:
A. The signal peptide of the DDDK protein, wherein the signal peptide has the amino acid sequence MFNLKTILISTLASIAVA (SEQ ID NO: 1).
B. The signal peptide according to embodiment A, wherein said signal peptide can be isolated from a *Pichia pastoris* strain.
C. The signal peptide according to embodiment A, wherein the amino acid sequence can be obtained by using a nucleic acid molecule comprising or consisting of codon, wherein any existing codon for a given amino acid can be present in said nucleic acid molecule.
D. A nucleic acid molecule comprising the nucleotide sequence of SEQ ID NO: 2.
E. A nucleic acid molecule comprising a nucleotide sequence encoding the amino acid sequence according to SEQ ID NO: 1, wherein said nucleic sequence has an identity of at least 80% to the nucleotide sequence of SEQ ID NO: 2.
F. A method of Kex2p-independent protein secretion using the signal peptide of SEQ ID NO: 1 the nucleotide sequence of SEQ ID NO: 2 and/or the nucleotide sequence having at least 80% identity to the nucleotide sequence of SEQ ID NO: 2.
G. A *Pichia pastoris* host cell comprising a PMT1 gene knock out and a nucleic acid molecule comprising the nucleotide sequence of SEQ ID NO: 2 or a nucleotide sequence having at least 80% identity to the nucleotide sequence of SEQ ID NO: 2.
H. Use of the signal peptide of the DDDK protein for heterologous protein expression in *P. pastoris.*
I. Porcine carboxypeptidase B expressed in *P. pastoris* using a nucleic acid molecule wherein nucleotide sequence encoding the porcine carboxypeptidase B is fused in frame the nucleic acid sequence encoding the *P. pastoris* DDDK signal peptide.
J. *Erythrina* Trypsin Inhibitor expressed in *P. pastoris* using a nucleic acid molecule wherein nucleotide sequence encoding the *Erythrina* Trypsin Inhibitor B is fused in frame the nucleic acid sequence encoding the *P. pastoris* DDDK signal peptide.

## Claims

1. A heterologous nucleic acid molecule comprising a nucleotide sequence encoding a polypeptide of interest and a nucleotide sequence encoding the *P. pastoris* signal amino acid sequence according to SEQ ID NO: 1, the nucleotide sequence encoding said polypeptide of interest being present contiguously (in frame) at and to the 3' end of the nucleotide sequence encoding the amino acid sequence according to SEQ ID NO: 1,
such that the nucleotide sequence encoding the amino acid sequence according to SEQ ID NO: 1 and the nucleotide sequence encoding said polypeptide of interest provide a continuous open reading frame for a heterologous polypeptide that consists of the amino acid sequence according to SEQ ID NO: 1 at its N-terminal end and the polypeptide of interest.

2. The heterologous nucleic acid molecule according to claim 1, wherein the nucleotide sequence encoding the amino acid sequence according to SEQ ID NO: 1 consists of the nucleotide sequence according to SEQ ID NO: 2.

3. The heterologous nucleic acid molecule according to claim 1 or 2, wherein said nucleic acid molecule further comprises a nucleotide sequence representing a promoter, said nucleotide sequence representing a promoter being present at a position within the heterologous nucleic acid molecule, such that the promoter controls the expression of the nucleotide sequence encoding the heterologous polypeptide.

4. The heterologous nucleic acid molecule according to claim 3, wherein the promoter is the AOX1 promoter.

5. A heterologous polypeptide consisting of a polypeptide of interest and the amino acid sequence according to SEQ ID NO: 1.

6. A host cell comprising a heterologous nucleic acid molecule as defined in any of claims 1 to 4 and/or expressing a heterologous polypeptide as defined in claim 5, wherein the host cell is a *Pichia pastoris* cell.

7. The host cell of claim 6, wherein the host cell is a host cell having its Kex2p gene disrupted.

8. Use of a heterologous nucleic acid molecule according to any of claims 1 to 4 for having a polypeptide of interest that is expressed in *Pichia pastoris* being secreted into a culture medium.

9. A method for producing a polypeptide, said method comprising the steps of:
a) transforming a *Pichia pastoris* host cell with a nucleic acid molecule, wherein the nucleotide sequence encoding a polypeptide of interest is contiguously fused to a nucleotide sequence encoding the DDDK signal peptide of SEQ ID NO: 1, such that a continuous open reading frame is provided which encodes a heterologous fusion protein consisting of the *P. pastoris* DDDK signal peptide and the polypeptide of interest; and
b) inducing expression of the heterologous fusion protein.

10. The method according to claim 9, further comprising isolating from the culture medium the polypeptide of interest, which was expressed as heterologous polypeptide in the host cell, wherein said host cell was cultivated during expression.

11. The method according to claim 9 or 10, wherein the host cell is a host cell having its Kex2p gene disrupted.

12. The method according to any of claims 9 to 11, wherein the expression of the heterologous fusion protein is under the control of the AOX 1 promoter.

13. The method according to any of claims 9 to 12, wherein the nucleotide sequence encoding the polypeptide of interest is a nucleotide sequence selected from the group of nucleotide sequences encoding a carboxypeptidase B and nucleotide sequences encoding an erythrina trypsin inhibitor.

## Patentansprüche

1. Heterologes Nukleinsäuremolekül, das eine Nukleotidsequenz aufweist, die für ein Polypeptid von Interesse sowie eine Nukleotidsequenz kodiert, die für die *P. pastoris-*Aminosäure-Signalsequenz gemäß SEQ ID NO: 1 kodiert, wobei die Nukleotidsequenz, die für das Polypeptid von Interesse kodiert, angrenzend (in frame) an das 3'-Ende der Nukleotidsequenz vorliegt, die für die Aminosäuresequenz gemäß SEQ ID NO: 1 kodiert,
so dass die Nukleotidsequenz, die die Aminosäuresequenz gemäß SEQ ID NO: 1 kodiert, und die Nukleotidsequenz, die das Polypeptid von Interesse kodiert, einen kontinuierlichen offenen Leserahmen für ein heterologes Polypeptid bereitstellen, das aus der Aminosäuresequenz gemäß SEQ ID NO: 1 an seinem N-terminalen Ende und dem Polypeptid von Interesse besteht.

2. Heterologes Nukleinsäuremolekül nach Anspruch 1, wobei die Nukleotidsequenz, die die Aminosäuresequenz nach SEQ ID NO: 1 codiert, aus der Nukleotidsequenz gemäß SEQ ID NO: 2 besteht.

3. Heterologes Nukleinsäuremolekül nach Anspruch 1 oder 2, wobei das besagte Nukleinsäuremolekül weiterhin eine Nukleotidsequenz aufweist, die einen Promotor darstellt, wobei die besagte Nukleotidsequenz, die einen Promotor darstellt, an einer Position innerhalb des heterologen Nukleinsäuremoleküls vorhanden ist, so dass der Promotor die Expression der das heterologe Polypeptid kodierenden Nukleotidsequenz steuert.

4. Heterologes Nukleinsäuremolekül nach Anspruch 3, wobei der Promotor der AOX1-Promotor ist.

5. Heterologes Polypeptid, bestehend aus einem Polypeptid von Interesse und der Aminosäuresequenz gemäß SEQ ID NO: 1.

6. Wirtszelle, die ein heterologes Nukleinsäuremolekül wie in einem der Ansprüche 1 bis 4 definiert aufweist und/oder ein heterologes wie in Anspruch 5 definiertes Polypeptid exprimiert, wobei die Wirtszelle eine *Pichia pastoris*-Zelle ist.

7. Wirtszelle nach Anspruch 6, wobei die Wirtszelle eine Wirtszelle ist, deren Kex2p-Gen unterbrochen ist.

8. Verwendung eines heterologen Nukleinsäuremoleküls gemäß einem der Ansprüche 1 bis 4, um ein Polypeptid von Interesse, das in *Pichia pastoris* exprimiert wird, in ein Kulturmedium sezernieren zu lassen.

9. Verfahren zur Herstellung eines Polypeptids, wobei das besagte Verfahren die Schritte aufweist:
a) eine *Pichia pastoris*-Wirtszelle mit einem Nukleinsäuremolekül transformieren, wobei die Nukleotidsequenz ein Polypeptid von Interesse kodiert, an eine Nukleotidsequenz angrenzend fusioniert ist, die für das DDDK-Signalpeptid von SEQ ID NO: 1 kodiert, so dass ein kontinuierlicher offener Leserahmen bereitgestellt wird, der für ein heterologes Fusionsprotein kodiert, das aus dem *P. pastoris* DDDK-Signalpeptid und dem Polypeptid von Interesse besteht, und
b) die Expression des heterologen Fusionsproteins induzieren.

10. Verfahren nach Anspruch 9, das weiterhin aufweist, das Polypeptid von Interesse, das als heterologes Polypeptid in der Wirtszelle exprimiert wurde, aus dem Kulturmedium zu isolieren, wobei die besagte Wirtszelle während der Expression kultiviert wurde.

11. Verfahren nach Anspruch 9 oder 10, wobei die Wirtszelle eine Wirtszelle ist, deren Kex2p-Gen unterbrochen ist.

12. Verfahren nach einem der Ansprüche 9 bis 11, wobei die Expression des heterologen Fusionsproteins unter der Kontrolle des AOX-1-Promotors steht.

13. Verfahren nach einem der Ansprüche 9 bis 12, wobei die das Polypeptid von Interesse kodierende Nukleotidsequenz eine Nukleotidsequenz ist, die aus der Gruppe von Nukleotidsequenzen, die Carboxypeptidase B kodieren und Nukleotidsequenzen, die einen Erythrina-Trypsin-Inhibitor kodieren, ausgewählt ist.

## Revendications

1. Molécule d'acide nucléique hétérologue comprenant une séquence de nucléotides codant un polypeptide d'intérêt et une séquence de nucléotides codant la séquence d'acides aminés de signal *P. pastoris* selon SEQ ID n° 1, la séquence de nucléotides codant ledit polypeptide d'intérêt étant présente de manière continue (dans le cadre) au niveau et sur l'extrémité 3' de la séquence de nucléotides codant la séquence d'acides aminés selon SEQ ID n° 1,
de sorte que la séquence de nucléotides codant la séquence d'acides aminés selon SEQ ID n° 1 et la séquence de nucléotides codant ledit polypeptide d'intérêt fournissent un cadre de lecture ouvert continu pour un polypeptide hétérologue qui consiste en la séquence d'acides aminés selon SEQ ID n° 1 au niveau de son extrémité à terminaison N et le polypeptide d'intérêt.

2. Molécule d'acide nucléique hétérologue selon la revendication 1, dans laquelle la séquence de nucléotides codant la séquence d'acides aminés selon SEQ ID n° 1 consiste en la séquence de nucléotides selon SEQ ID n° 2.

3. Molécule d'acide nucléique hétérologue selon la revendication 1 ou 2, dans laquelle ladite molécule d'acide nucléique comprend en outre une séquence de nucléotides représentant un promoteur, ladite séquence de nucléotides représentant un promoteur étant présente au niveau d'une position à l'intérieur de la molécule d'acide nucléique hétérologue, de sorte que le promoteur commande l'expression de la séquence de nucléotides codant le polypeptide hétérologue.

4. Molécule d'acide nucléique hétérologue selon la revendication 3, dans laquelle le promoteur est le promoteur AOX1.

5. Polypeptide hétérologue consistant en un polypeptide d'intérêt et la séquence d'acides aminés selon SEQ ID n° 1.

6. Cellule hôte comprenant une molécule d'acide nucléique hétérologue telle que définie selon l'une quelconque des revendications 1 à 4 et/ou exprimant un polypeptide hétérologue tel que défini dans la revendication 5, dans laquelle la cellule hôte est une cellule *Pichia pastoris.*

7. Cellule hôte selon la revendication 6, dans laquelle la cellule hôte est une cellule hôte ayant son gène Kex2p perturbé.

8. Utilisation d'une molécule d'acide nucléique hétérologue selon l'une quelconque des revendications 1 à 4, pour avoir un polypeptide d'intérêt qui est exprimé dans *Pichia pastoris* qui est sécrétée dans un milieu de culture.

9. Procédé pour produire un polypeptide, ledit procédé comprenant les étapes de :
a) transformation d'une cellule hôte *Pichia pastoris* avec une molécule d'acide nucléique, dans laquelle la séquence de nucléotides codant un polypeptide d'intérêt est fusionnée de façon continue à une séquence de nucléotides codant le peptide de signal DDDK de SEQ ID n° 1, de sorte qu'un cadre de lecture ouvert continu est prévu qui code une protéine de fusion hétérologue constituée par le peptide de signal DDDK de *P. pastoris* et le polypeptide d'intérêt ; et
b) incitation de l'expression de la protéine de fusion hétérologue.

10. Procédé selon la revendication 9, comprenant en outre l'isolement à partir du milieu de culture du polypeptide d'intérêt, qui a été exprimé en tant que polypeptide hétérologue dans la cellule hôte, dans lequel ladite cellule hôte a été cultivée pendant l'expression.

11. Procédé selon la revendication 9 ou 10, dans lequel la cellule hôte est une cellule hôte ayant son gène Kex2p perturbé.

12. Procédé selon l'une quelconque des revendications 9 à 11, dans lequel l'expression de la protéine de fusion hétérologue est sous la commande du promoteur AOX1.

13. Procédé selon l'une quelconque des revendications 9 à 12, dans lequel la séquence de nucléotides codant le polypeptide d'intérêt est une séquence de nucléotides sélectionnée à partir du groupe de séquences de nucléotides codant une carboxypeptidase B et de séquences de nucléotides codant un inhibiteur de trypsine erythrina.


### SEQUENCE LISTING

<110> BIOCON Limited
<120> SIGNAL SEQUENCE FOR PROTEIN EXPRESSION IN PICHIA PASTORIS
<130> unknown
<160> 27
<170> PatentIn version 3.3
<210> 1
   <211> 18
   <212> PRT
   <213> Pichia pastoris
<400> 1
   Met Phe Asn Leu Lys Thr Ile Leu Ile Ser Thr Leu Ala Ser Ile Ala
   1               5                   10                  15
   Val Ala
<210> 2
   <211> 54
   <212> DNA
   <213> Pichia pastoris
<400> 2
   atgttcaacc tgaaaactat tctcatctca acacttgcat cgatcgctgt tgcc 54
<210> 3
   <211> 12
   <212> PRT
   <213> Pichia pastoris
<400> 3
   Asp Gln Thr Phe Gly Val Leu Leu Ile Arg Ser Gly
   1               5                   10
<210> 4
   <211> 289
   <212> PRT
   <213> Pichia pastoris
<400> 4
   Met Phe Asn Leu Lys Thr Ile Leu Ile Ser Thr Leu Ala Ser Ile Ala
   1               5                   10                  15
   Val Ala Asp Gln Thr Phe Gly Val Leu Leu Ile Arg Ser Gly Ser Pro
               20                  25                  30
   Tyr His Tyr Ser Thr Leu Thr Asn Arg Asp Glu Lys Ile Val Ala Gly
               35                  40                  45
   Gly Gly Asn Lys Lys Val Thr Leu Thr Asp Glu Gly Ala Leu Lys Tyr
       50                  55                  60
   Asp Gly Gly Lys Trp Ile Gly Leu Asp Asp Asp Gly Tyr Ala Val Gln
   65                  70                  75                  80
   Thr Asp Lys Pro Val Thr Gly Trp Ser Thr Asn Gly Gly Tyr Leu Tyr
                   85                  90                  95
   Phe Asp Gln Gly Leu Ile Val Cys Thr Glu Asp Tyr Ile Gly Tyr Val
               100                 105                 110
   Lys Lys His Gly Glu Cys Lys Gly Asp Ser Tyr Gly Met Ala Trp Lys
           115                 120                 125
   Val Leu Pro Ala Asp Asp Asp Lys Asp Asp Asp Lys Asp Asp Asp Lys
       130                 135                 140
   Asp Asp Asp Lys Asp Tyr Asp Asp Asp Asn Asp His Gly Asp Gly Asp
   145                 150                 155                 160
   Tyr Tyr Cys Ser Ile Thr Gly Thr Tyr Ala Ile Lys Ser Lys Gly Ser
                   165                 170                 175
   Lys His Gln Tyr Glu Ala Ile Lys Lys Val Asp Ala His Pro His Val
               180                 185                 190
   Phe Ser Val Gly Gly Asp Gln Gly Asn Asp Leu Ile Val Thr Phe Gln
           195                 200                 205
   Lys Asp Cys Ser Leu Val Asp Gln Asp Asn Arg Gly Tyr Val Tyr Asp
       210                 215                 220
   Pro Asn Ser Gly Glu Val Gly Asn Val Asp Pro Trp Gly Glu Leu Thr
   225                 230                 235                 240
   Pro Ser Val Lys Trp Asp Ile Asp Asp Gly Tyr Leu Ile Phe Asn Gly
                   245                 250                 255
   Glu Ser Asn Phe Arg Ser Cys Pro Ser Gly Asn Gly Tyr Ser Leu Ser
               260                 265                 270
   Ile Lys Asp Cys Val Gly Gly Thr Asp Ile Gly Leu Lys Val Trp Glu
           275                 280                 285
   Lys
<210> 5
   <211> 271
   <212> PRT
   <213> Pichia pastoris
<400> 5
   Asp Gln Thr Phe Gly Val Leu Leu Ile Arg Ser Gly Ser Pro Tyr His
   1               5                   10                  15
   Tyr Ser Thr Leu Thr Asn Arg Asp Glu Lys Ile Val Ala Gly Gly Gly
               20                  25                  30
   Asn Lys Lys Val Thr Leu Thr Asp Glu Gly Ala Leu Lys Tyr Asp Gly
           35                  40                  45
   Gly Lys Trp Ile Gly Leu Asp Asp Asp Gly Tyr Ala Val Gln Thr Asp
       50                  55                  60
   Lys Pro Val Thr Gly Trp Ser Thr Asn Gly Gly Tyr Leu Tyr Phe Asp
   65                  70                  75                  80
   Gln Gly Leu Ile Val Cys Thr Glu Asp Tyr Ile Gly Tyr Val Lys Lys
                   85                  90                  95
   His Gly Glu Cys Lys Gly Asp Ser Tyr Gly Met Ala Trp Lys Val Leu
               100                 105                 110
   Pro Ala Asp Asp Asp Lys Asp Asp Asp Lys Asp Asp Asp Lys Asp Asp
           115                 120                 125
   Asp Lys Asp Tyr Asp Asp Asp Asn Asp His Gly Asp Gly Asp Tyr Tyr
       130                 135                 140
   Cys Ser Ile Thr Gly Thr Tyr Ala Ile Lys Ser Lys Gly Ser Lys His
   145                 150                 155                 160
   Gln Tyr Glu Ala Ile Lys Lys Val Asp Ala His Pro His Val Phe Ser
                   165                 170                 175
   Val Gly Gly Asp Gln Gly Asn Asp Leu Ile Val Thr Phe Gln Lys Asp
               180                 185                 190
   Cys Ser Leu Val Asp Gln Asp Asn Arg Gly Tyr Val Tyr Asp Pro Asn
           195                 200                 205
   Ser Gly Glu Val Gly Asn Val Asp Pro Trp Gly Glu Leu Thr Pro Ser
       210                 215                 220
   Val Lys Trp Asp Ile Asp Asp Gly Tyr Leu Ile Phe Asn Gly Glu Ser
   225                 230                 235                 240
   Asn Phe Arg Ser Cys Pro Ser Gly Asn Gly Tyr Ser Leu Ser Ile Lys
                   245                 250                 255
   Asp Cys Val Gly Gly Thr Asp Ile Gly Leu Lys Val Trp Glu Lys
               260                 265                 270
<210> 6
   <211> 32
   <212> DNA
   <213> Artificial
<220>
   <223> forward primer for amplification of P. pastoris DDDK open reading frame.
<400> 6
   cgggatccat gttcaacctg aaaactattc tc 32
<210> 7
   <211> 35
   <212> DNA
   <213> Artificial
<220>
   <223> reverse primer for amplification of P. pastoris DDDK open reading frame.
<400> 7
   cggaattctt atttctccca tactttaagg ccaat 35
<210> 8
   <211> 29
   <212> DNA
   <213> Artificial
<220>
   <223> forward primer for amplification of a truncated DDDK open reading frame.
<400> 8
   gaagatctga ccaaaccttc ggtgtcctt 29
<210> 9
   <211> 40
   <212> DNA
   <213> Artificial
<220>
   <223> reverse primer for amplifying the P. pastoris DDDK gene.
<400> 9
   atcgatcgct gttgccaaaa gagaccaaac cttcggtgtc 40
<210> 10
   <211> 23
   <212> DNA
   <213> Artificial
<220>
   <223> forward primer for amplifying the P. pastoris DDDK signal sequence.
<400> 10
   ggatccatgt tcaacctgaa aac 23
<210> 11
   <211> 30
   <212> DNA
   <213> Artificial
<220>
   <223> forward primer for amplifying a truncated P. pastoris DDDK signal seqeunce.
<400> 11
   tattctcatc tcaacacttg catcgatcgc 30
<210> 12
   <211> 16
   <212> DNA
   <213> Artificial
<220>
   <223> forward primer for amplifying a truncated P. pastoris DDDK signal seqeunce.
<400> 12
   tgttgccctc gagcgg 16
<210> 13
   <211> 30
   <212> DNA
   <213> Artificial
<220>
   <223> reverse primer for amplifying the P. pastoris DDDK signal sequence
<400> 13
   ccgctcgagg gcaacagcga tcgatgcaag 30
<210> 14
   <211> 29
   <212> DNA
   <213> Artificial
<220>
   <223> reverse primer for amplifying a truncated P. pastoris DDDK signal sequence.
<400> 14
   tgttgagatg agaatagttt tcaggttga 29
<210> 15
   <211> 14
   <212> DNA
   <213> Artificial
<220>
   <223> reverse primer for amplifying a truncated P. pastoris DDDK signal sequence.
<400> 15
   acatatggaa ttcc 14
<210> 16
   <211> 38
   <212> DNA
   <213> Artificial
<220>
   <223> forward primer for amplifying the CPB coding seqeunce.
<400> 16
   ccatcgatcg ctgttgccga agctgaggca catcactc 38
<210> 17
   <211> 38
   <212> DNA
   <213> Artificial
<220>
   <223> forward primer for amplifying the ETI coding sequence.
<400> 17
   ccatcgatcg ctgttgccgt tttgttggat ggtaatgg 38
<210> 18
   <211> 20
   <212> DNA
   <213> Artificial
<220>
   <223> reverse primer
<400> 18
   tgcccaactt gaactgagga 20
<210> 19
   <211> 946
   <212> DNA
   <213> Pichia pastoris
<400> 19
   agatctaaca tccaaagacg aaaggttgaa tgaaaccttt ttgccatccg acatccacag 60
<210> 20
   <211> 1221
   <212> DNA
   <213> Sus sp.
<400> 20
   atggaagctg aggcacatca ctctggtgaa cattttgaag gagagaaagt tttcagagtt      60
   aatgttgaag atgagaacga catttctttg cttcatgagt tggcctccac tagacaaatt     120
   gacttctgga agccagattc agtcactcaa atcaaaccac actctaccgt tgactttaga     180
   gttaaggctg aggatatttt ggctgttgag gactttctgg aacagaatga gttgcaatac     240
   gaggttttga tcaacaattt gagatctgtt cttgaggctc aatttgactc tagagtcaga     300
   acaactggac attcctacga gaagtataat aactgggaga ctattgaagc ctggactaaa     360
   caagtcactt cagagaatcc tgatctgatt tctagaactg ccattggtac cacatttctg     420
   ggtaacaata tctacttgct gaaagttggt aagcctggac ctaataagcc agctatcttt     480
   atggattgtg gatttcatgc tagagagtgg atttctcatg cattctgtca atggtttgtt     540
   agagaggctg ttttgactta tggatacgag tctcacatga ctgagtttct taacaagttg     600
   gacttctacg ttttgccagt tttgaacatt gatggttaca tctacacttg gactaagaat     660
   agaatgtgga gaaagactag atccactaat gctggtacaa cttgtattgg tactgatcca     720
   aacagaaact ttgacgctgg atggtgtact accggtgcct caactgatcc atgtgacgag     780
   acttactgtg gttctgctgc cgagtctgag aaagagacta aagctttggc tgactttatc     840
   agaaataact tgtcatccat taaagcttac ttgactattc attcttattc tcaaatgatt     900
   ttgtatccat actcctacga ttacaagttg cctgagaaca atgcagagtt gaataacctg     960
   gctaaggctg ccgtcaaaga gcttgccact ttgtacggta ctaagtacac ttacggtcca    1020
   ggtgctacta ccatttaccc agctgcaggt ggatctgatg actgggcata cgatcaaggt    1080
   atcaagtact cttttacctt tgagttgaga gacaaaggta gatacggatt tatcttgcct    1140
   gagtctcaaa ttcaagctac ttgtgaagag actatgttgg ctattaagta cgtcactaac    1200
   tacgttttgg gacacttgta a                                               1221
<210> 21
   <211> 406
   <212> PRT
   <213> Sus sp.
<400> 21
   Met Glu Ala Glu Ala His His Ser Gly Glu His Phe Glu Gly Glu Lys
   1               5                   10                  15
   Val Phe Arg Val Asn Val Glu Asp Glu Asn Asp Ile Ser Leu Leu His
               20                  25                  30
   Glu Leu Ala Ser Thr Arg Gln Ile Asp Phe Trp Lys Pro Asp Ser Val
           35                  40                  45
   Thr Gln Ile Lys Pro His Ser Thr Val Asp Phe Arg Val Lys Ala Glu
       50                  55                  60
   Asp Ile Leu Ala Val Glu Asp Phe Leu Glu Gln Asn Glu Leu Gln Tyr
   65                  70                  75                  80
   Glu Val Leu Ile Asn Asn Leu Arg Ser Val Leu Glu Ala Gln Phe Asp
                   85                  90                  95
   Ser Arg Val Arg Thr Thr Gly His Ser Tyr Glu Lys Tyr Asn Asn Trp
               100                 105                 110
   Glu Thr Ile Glu Ala Trp Thr Lys Gln Val Thr Ser Glu Asn Pro Asp
           115                 120                 125
   Leu Ile Ser Arg Thr Ala Ile Gly Thr Thr Phe Leu Gly Asn Asn Ile
       130                 135                 140
   Tyr Leu Leu Lys Val Gly Lys Pro Gly Pro Asn Lys Pro Ala Ile Phe
   145                 150                 155                 160
   Met Asp Cys Gly Phe His Ala Arg Glu Trp Ile Ser His Ala Phe Cys
                   165                 170                 175
   Gln Trp Phe Val Arg Glu Ala Val Leu Thr Tyr Gly Tyr Glu Ser His
               180                 185                 190
   Met Thr Glu Phe Leu Asn Lys Leu Asp Phe Tyr Val Leu Pro Val Leu
           195                 200                 205
   Asn Ile Asp Gly Tyr Ile Tyr Thr Trp Thr Lys Asn Arg Met Trp Arg
       210                 215                 220
   Lys Thr Arg Ser Thr Asn Ala Gly Thr Thr Cys Ile Gly Thr Asp Pro
   225                 230                 235                 240
   Asn Arg Asn Phe Asp Ala Gly Trp Cys Thr Thr Gly Ala Ser Thr Asp
                   245                 250                 255
   Pro Cys Asp Glu Thr Tyr Cys Gly Ser Ala Ala Glu Ser Glu Lys Glu
               260                 265                 270
   Thr Lys Ala Leu Ala Asp Phe Ile Arg Asn Asn Leu Ser Ser Ile Lys
           275                 280                 285
   Ala Tyr Leu Thr Ile His Ser Tyr Ser Gln Met Ile Leu Tyr Pro Tyr
       290                 295                 300
   Ser Tyr Asp Tyr Lys Leu Pro Glu Asn Asn Ala Glu Leu Asn Asn Leu
   305                 310                 315                 320
   Ala Lys Ala Ala Val Lys Glu Leu Ala Thr Leu Tyr Gly Thr Lys Tyr
                   325                 330                 335
   Thr Tyr Gly Pro Gly Ala Thr Thr Ile Tyr Pro Ala Ala Gly Gly Ser
               340                 345                 350
   Asp Asp Trp Ala Tyr Asp Gln Gly Ile Lys Tyr Ser Phe Thr Phe Glu
           355                 360                 365
   Leu Arg Asp Lys Gly Arg Tyr Gly Phe Ile Leu Pro Glu Ser Gln Ile
       370                 375                 380
   Gln Ala Thr Cys Glu Glu Thr Met Leu Ala Ile Lys Tyr Val Thr Asn
   385                 390                 395                 400
   Tyr Val Leu Gly His Leu
                   405
<210> 22
   <211> 1272
   <212> DNA
   <213> Artificial
<220>
   <223> coding sequence for porcine CPB comprising the P. pastoris DDDK signal sequence.
<400> 22
   atgttcaacc tgaaaactat tctcatctca acacttgcat cgatcgctgt tgccgaagct      60
   gaggcacatc actctggtga acattttgaa ggagagaaag ttttcagagt taatgttgaa     120
   gatgagaacg acatttcttt gcttcatgag ttggcctcca ctagacaaat tgacttctgg     180
   aagccagatt cagtcactca aatcaaacca cactctaccg ttgactttag agttaaggct     240
   gaggatattt tggctgttga ggactttctg gaacagaatg agttgcaata cgaggttttg     300
   atcaacaatt tgagatctgt tcttgaggct caatttgact ctagagtcag aacaactgga     360
   cattcctacg agaagtataa taactgggag actattgaag cctggactaa acaagtcact     420
   tcagagaatc ctgatctgat ttctagaact gccattggta ccacatttct gggtaacaat     480
   atctacttgc tgaaagttgg taagcctgga cctaataagc cagctatctt tatggattgt     540
   ggatttcatg ctagagagtg gatttctcat gcattctgtc aatggtttgt tagagaggct     600
   gttttgactt atggatacga gtctcacatg actgagtttc ttaacaagtt ggacttctac     660
   gttttgccag ttttgaacat tgatggttac atctacactt ggactaagaa tagaatgtgg     720
   agaaagacta gatccactaa tgctggtaca acttgtattg gtactgatcc aaacagaaac     780
   tttgacgctg gatggtgtac taccggtgcc tcaactgatc catgtgacga gacttactgt     840
   ggttctgctg ccgagtctga gaaagagact aaagctttgg ctgactttat cagaaataac     900
   ttgtcatcca ttaaagctta cttgactatt cattcttatt ctcaaatgat tttgtatcca     960
   tactcctacg attacaagtt gcctgagaac aatgcagagt tgaataacct ggctaaggct    1020
   gccgtcaaag agcttgccac tttgtacggt actaagtaca cttacggtcc aggtgctact    1080
   accatttacc cagctgcagg tggatctgat gactgggcat acgatcaagg tatcaagtac    1140
   tcttttacct ttgagttgag agacaaaggt agatacggat ttatcttgcc tgagtctcaa    1200
   attcaagcta cttgtgaaga gactatgttg gctattaagt acgtcactaa ctacgttttg    1260
   ggacacttgt aa                                                        1272
<210> 23
   <211> 423
   <212> PRT
   <213> Artificial
<220>
   <223> porcine carboxypeptidase B comprising the P. pastoris DDDK signal sequence.
<400> 23
   Met Phe Asn Leu Lys Thr Ile Leu Ile Ser Thr Leu Ala Ser Ile Ala
   1               5                   10                  15
   Val Ala Glu Ala Glu Ala His His Ser Gly Glu His Phe Glu Gly Glu
               20                  25                  30
   Lys Val Phe Arg Val Asn Val Glu Asp Glu Asn Asp Ile Ser Leu Leu
           35                  40                  45
   His Glu Leu Ala Ser Thr Arg Gln Ile Asp Phe Trp Lys Pro Asp Ser
       50                  55                  60
   Val Thr Gln Ile Lys Pro His Ser Thr Val Asp Phe Arg Val Lys Ala
   65                  70                  75                  80
   Glu Asp Ile Leu Ala Val Glu Asp Phe Leu Glu Gln Asn Glu Leu Gln
                   85                  90                  95
   Tyr Glu Val Leu Ile Asn Asn Leu Arg Ser Val Leu Glu Ala Gln Phe
               100                 105                 110
   Asp Ser Arg Val Arg Thr Thr Gly His Ser Tyr Glu Lys Tyr Asn Asn
           115                 120                 125
   Trp Glu Thr Ile Glu Ala Trp Thr Lys Gln Val Thr Ser Glu Asn Pro
       130                 135                 140
   Asp Leu Ile Ser Arg Thr Ala Ile Gly Thr Thr Phe Leu Gly Asn Asn
   145                 150                 155                 160
   Ile Tyr Leu Leu Lys Val Gly Lys Pro Gly Pro Asn Lys Pro Ala Ile
                   165                 170                 175
   Phe Met Asp Cys Gly Phe His Ala Arg Glu Trp Ile Ser His Ala Phe
               180                 185                 190
   Cys Gln Trp Phe Val Arg Glu Ala Val Leu Thr Tyr Gly Tyr Glu Ser
           195                 200                 205
   His Met Thr Glu Phe Leu Asn Lys Leu Asp Phe Tyr Val Leu Pro Val
       210                 215                 220
   Leu Asn Ile Asp Gly Tyr Ile Tyr Thr Trp Thr Lys Asn Arg Met Trp
   225                 230                 235                 240
   Arg Lys Thr Arg Ser Thr Asn Ala Gly Thr Thr Cys Ile Gly Thr Asp
                   245                 250                 255
   Pro Asn Arg Asn Phe Asp Ala Gly Trp Cys Thr Thr Gly Ala Ser Thr
               260                 265                 270
   Asp Pro Cys Asp Glu Thr Tyr Cys Gly Ser Ala Ala Glu Ser Glu Lys
           275                 280                 285
   Glu Thr Lys Ala Leu Ala Asp Phe Ile Arg Asn Asn Leu Ser Ser Ile
       290                 295                 300
   Lys Ala Tyr Leu Thr Ile His Ser Tyr Ser Gln Met Ile Leu Tyr Pro
   305                 310                 315                 320
   Tyr Ser Tyr Asp Tyr Lys Leu Pro Glu Asn Asn Ala Glu Leu Asn Asn
                   325                 330                 335
   Leu Ala Lys Ala Ala Val Lys Glu Leu Ala Thr Leu Tyr Gly Thr Lys
               340                 345                 350
   Tyr Thr Tyr Gly Pro Gly Ala Thr Thr Ile Tyr Pro Ala Ala Gly Gly
           355                 360                 365
   Ser Asp Asp Trp Ala Tyr Asp Gln Gly Ile Lys Tyr Ser Phe Thr Phe
       370                 375                 380
   Glu Leu Arg Asp Lys Gly Arg Tyr Gly Phe Ile Leu Pro Glu Ser Gln
   385                 390                 395                 400
   Ile Gln Ala Thr Cys Glu Glu Thr Met Leu Ala Ile Lys Tyr Val Thr
                   405                 410                 415
   Asn Tyr Val Leu Gly His Leu
               420
<210> 24
   <211> 522
   <212> DNA
   <213> Erythrina sp.
<400> 24
   atggttttgt tggatggtaa tggtgaagtt gttcaaaatg gtggtactta ttatttgttg     60
   ccacaagttt gggctcaagg tggtggtgtt caattggcta agactggtga agaaacttgt    120
   ccattgactg ttgttcaatc tccaaatgaa ttgtctgatg gtaagccaat tagaattgaa    180
   tctagattga gatctgcttt tattccagat gatgataagg ttagaattgg ttttgcttat    240
   gctccaaagt gtgctccatc tccatggtgg actgttgttg aagatgaaca agaaggtttg    300
   tctgttaagt tgtctgaaga tgaatctact caatttgatt atccatttaa gtttgaacaa    360
   gtttctgatc aattgcattc ttataagttg ttgtattgtg aaggtaagca tgaaaagtgt    420
   gcttctattg gtattaatag agatcaaaag ggttatagaa gattggttgt tactgaagat    480
   tatccattga ctgttgtttt gaagaaggat gaatcttctt aa                       522
<210> 25
   <211> 173
   <212> PRT
   <213> Erythrina sp.
<400> 25
   Met Val Leu Leu Asp Gly Asn Gly Glu Val Val Gln Asn Gly Gly Thr
   1               5                   10                  15
   Tyr Tyr Leu Leu Pro Gln Val Trp Ala Gln Gly Gly Gly Val Gln Leu
               20                  25                  30
   Ala Lys Thr Gly Glu Glu Thr Cys Pro Leu Thr Val Val Gln Ser Pro
           35                  40                  45
   Asn Glu Leu Ser Asp Gly Lys Pro Ile Arg Ile Glu Ser Arg Leu Arg
       50                  55                  60
   Ser Ala Phe Ile Pro Asp Asp Asp Lys Val Arg Ile Gly Phe Ala Tyr
   65                  70                  75                  80
   Ala Pro Lys Cys Ala Pro Ser Pro Trp Trp Thr Val Val Glu Asp Glu
                   85                  90                  95
   Gln Glu Gly Leu Ser Val Lys Leu Ser Glu Asp Glu Ser Thr Gln Phe
               100                 105                 110
   Asp Tyr Pro Phe Lys Phe Glu Gln Val Ser Asp Gln Leu His Ser Tyr
           115                 120                 125
   Lys Leu Leu Tyr Cys Glu Gly Lys His Glu Lys Cys Ala Ser Ile Gly
       130                 135                 140
   Ile Asn Arg Asp Gln Lys Gly Tyr Arg Arg Leu Val Val Thr Glu Asp
   145                 150                 155                 160
   Tyr Pro Leu Thr Val Val Leu Lys Lys Asp Glu Ser Ser
                   165                 170
<210> 26
   <211> 573
   <212> DNA
   <213> Artificial
<220>
   <223> coding sequence for Erythrina Trypsin Inhibito comprising the P. pastoris DDDK signal sequence.
<400> 26
   atgttcaacc tgaaaactat tctcatctca acacttgcat cgatcgctgt tgccgttttg      60
   ttggatggta atggtgaagt tgttcaaaat ggtggtactt attatttgtt gccacaagtt     120
   tgggctcaag gtggtggtgt tcaattggct aagactggtg aagaaacttg tccattgact     180
   gttgttcaat ctccaaatga attgtctgat ggtaagccaa ttagaattga atctagattg     240
   agatctgctt ttattccaga tgatgataag gttagaattg gttttgctta tgctccaaag     300
   tgtgctccat ctccatggtg gactgttgtt gaagatgaac aagaaggttt gtctgttaag     360
   ttgtctgaag atgaatctac tcaatttgat tatccattta agtttgaaca agtttctgat     420
   caattgcatt cttataagtt gttgtattgt gaaggtaagc atgaaaagtg tgcttctatt     480
   ggtattaata gagatcaaaa gggttataga agattggttg ttactgaaga ttatccattg     540
   actgttgttt tgaagaagga tgaatcttct taa                                  573
<210> 27
   <211> 537
   <212> PRT
   <213> Artificial
<220>
   <223> Erythrina Trypsin Inhibitor comprising the P. pastoris DDDK signal sequence.
<400> 27
   Met Phe Asn Leu Lys Thr Ile Leu Ile Ser Thr Leu Ala Ser Ile Ala
   1               5                   10                  15
   Val Ala Gly Thr Thr Thr Thr Gly Thr Thr Gly Gly Ala Thr Gly Gly
               20                  25                  30
   Thr Ala Ala Thr Gly Gly Thr Gly Ala Ala Gly Thr Thr Gly Thr Thr
           35                  40                  45
   Cys Ala Ala Ala Ala Thr Gly Gly Thr Gly Gly Thr Ala Cys Thr Thr
       50                  55                  60
   Ala Thr Thr Ala Thr Thr Thr Gly Thr Thr Gly Cys Cys Ala Cys Ala
   65                  70                  75                  80
   Ala Gly Thr Thr Thr Gly Gly Gly Cys Thr Cys Ala Ala Gly Gly Thr
                   85                  90                  95
   Gly Gly Thr Gly Gly Thr Gly Thr Thr Cys Ala Ala Thr Thr Gly Gly
               100                 105                 110
   Cys Thr Ala Ala Gly Ala Cys Thr Gly Gly Thr Gly Ala Ala Gly Ala
           115                 120                 125
   Ala Ala Cys Thr Thr Gly Thr Cys Cys Ala Thr Thr Gly Ala Cys Thr
       130                 135                 140
   Gly Thr Thr Gly Thr Thr Cys Ala Ala Thr Cys Thr Cys Cys Ala Ala
   145                 150                 155                 160
   Ala Thr Gly Ala Ala Thr Thr Gly Thr Cys Thr Gly Ala Thr Gly Gly
                   165                 170                 175
   Thr Ala Ala Gly Cys Cys Ala Ala Thr Thr Ala Gly Ala Ala Thr Thr
               180                 185                 190
   Gly Ala Ala Thr Cys Thr Ala Gly Ala Thr Thr Gly Ala Gly Ala Thr
           195                 200                 205
   Cys Thr Gly Cys Thr Thr Thr Thr Ala Thr Thr Cys Cys Ala Gly Ala
       210                 215                 220
   Thr Gly Ala Thr Gly Ala Thr Ala Ala Gly Gly Thr Thr Ala Gly Ala
   225                 230                 235                 240
   Ala Thr Thr Gly Gly Thr Thr Thr Thr Gly Cys Thr Thr Ala Thr Gly
                   245                 250                 255
   Cys Thr Cys Cys Ala Ala Ala Gly Thr Gly Thr Gly Cys Thr Cys Cys
               260                 265                 270
   Ala Thr Cys Thr Cys Cys Ala Thr Gly Gly Thr Gly Gly Ala Cys Thr
           275                 280                 285
   Gly Thr Thr Gly Thr Thr Gly Ala Ala Gly Ala Thr Gly Ala Ala Cys
       290                 295                 300
   Ala Ala Gly Ala Ala Gly Gly Thr Thr Thr Gly Thr Cys Thr Gly Thr
   305                 310                 315                 320
   Thr Ala Ala Gly Thr Thr Gly Thr Cys Thr Gly Ala Ala Gly Ala Thr
                   325                 330                 335
   Gly Ala Ala Thr Cys Thr Ala Cys Thr Cys Ala Ala Thr Thr Thr Gly
               340                 345                 350
   Ala Thr Thr Ala Thr Cys Cys Ala Thr Thr Thr Ala Ala Gly Thr Thr
           355                 360                 365
   Thr Gly Ala Ala Cys Ala Ala Gly Thr Thr Thr Cys Thr Gly Ala Thr
       370                 375                 380
   Cys Ala Ala Thr Thr Gly Cys Ala Thr Thr Cys Thr Thr Ala Thr Ala
   385                 390                 395                 400
   Ala Gly Thr Thr Gly Thr Thr Gly Thr Ala Thr Thr Gly Thr Gly Ala
                   405                 410                 415
   Ala Gly Gly Thr Ala Ala Gly Cys Ala Thr Gly Ala Ala Ala Ala Gly
               420                 425                 430
   Thr Gly Thr Gly Cys Thr Thr Cys Thr Ala Thr Thr Gly Gly Thr Ala
           435                 440                 445
   Thr Thr Ala Ala Thr Ala Gly Ala Gly Ala Thr Cys Ala Ala Ala Ala
       450                 455                 460
   Gly Gly Gly Thr Thr Ala Thr Ala Gly Ala Ala Gly Ala Thr Thr Gly
   465                 470                 475                 480
   Gly Thr Thr Gly Thr Thr Ala Cys Thr Gly Ala Ala Gly Ala Thr Thr
                   485                 490                 495
   Ala Thr Cys Cys Ala Thr Thr Gly Ala Cys Thr Gly Thr Thr Gly Thr
               500                 505                 510
   Thr Thr Thr Gly Ala Ala Gly Ala Ala Gly Gly Ala Thr Gly Ala Ala
           515                 520                 525
   Thr Cys Thr Thr Cys Thr Thr Ala Ala
       530                 535